# DEMANDE DE BREVET EUROPEEN

(11) **EP 4 186 825 A2**
(43) Date de publication de la demande: **31.05.2023**
(21) Numéro de dépôt: 22209793.3
(22) Date de dépôt: 28.11.2022
(51) Int. Cl.: B65G 15/12, B65G 15/62, B65G 21/20

(54) **CONVOYEUR DE TRANSPORT D'OPERCULES ET MACHINE DE TRANSPORT ET DE DECONTAMINATION**

(30) Priorité: 30.11.2021 FR 2112743
(71) Demandeur: SYNERLINK, 95650 Puiseux-Pontoise (FR)
(72) Inventeur: MASSOT, Patrick, 72380 JOUE L'ABBE (FR); GROGNET, Stéphane, 95600 EAUBONNE (FR); DANDY, Laurent, 78700 CONFLANS SAINTE HONORINE (FR); DESBLEDS, Nathanael, 95660 CHAMPAGNE SUR OISE (FR)
(74) Mandataire: Derambure Conseil

(57) **Abrégé**

L'invention concerne un convoyeur de transport d'opercules comprenant un châssis, une première poulie (6) et une deuxième poulie portées par le châssis, un dispositif d'entrainement en rotation de la première poulie, une bande transporteuse sans fin montées entre la première poulie (6) et la deuxième poulie, un profilé de glissement (44, 46, 48, 50) porté par le châssis, le profilé de glissement comportant une rainure d'aspiration (56) débouchant au droit d'une face principale (53) de la bande transporteuse, une pompe adaptée pour aspirer de l'air au travers de ladite rainure d'aspiration,
ladite bande transporteuse comportant un orifice traversant positionné au droit de la rainure d'aspiration (56), lors du déplacement de la bande transporteuse ; un opercule étant maintenu par aspiration en contact d'une face de la bande transporteuse opposée à la face principale, lorsque l'orifice est en face de la rainure d'aspiration.

## Description

### Domaine technique de l'invention

La présente invention concerne un convoyeur de transport d'opercules et une machine de transport et de décontamination comprenant un tel convoyeur.

### Etat de la technique antérieure

Il est connu des convoyeurs de transport d'opercules dans lesquels les opercules sont positionnés et maintenus sur des plaques équipées de pions. Ces systèmes ont l'inconvénient d'être dédiés à un seul format d'opercule et doivent être changés à chaque changement de format d'opercule.

### Présentation de l'invention

Un premier but de la présente invention est de proposer un convoyeur capable de transporter des opercules de différents formats sans changement d'outillage dans la partie convoyage.

Un deuxième but de la présente invention est de proposer un convoyeur qui ne nécessite pas l'utilisation de plaques à pions pour maintenir les opercules, qui sont dédiées au format de l'opercule.

Un troisième but de la présente invention est de proposer une machine qui permet de décontaminer les opercules tout en les transportant.

Un quatrième but de la présente invention est de proposer un convoyeur plus hygiénique.

Un cinquième but est de proposer un convoyeur plus ergonomique.

### Résumé de l'invention

La présente invention a pour objet un convoyeur de transport d'opercules comprenant :
- un châssis,
- au moins une première poulie et une deuxième poulie portées par le châssis,
- un dispositif d'entrainement en rotation propre à entrainer en rotation au moins la première poulie,
- au moins une bande transporteuse sans fin montées entre la première poulie et la deuxième poulie, la bande transporteuse s'étendant selon une direction longitudinale, la bande transporteuse étant entrainée en déplacement par la première poulie,
- au moins un profilé de glissement porté par le châssis, le profilé de glissement comportant une première rainure d'aspiration s'étendant selon la direction longitudinale la première rainure d'aspiration débouchant au droit d'une face principale de la bande transporteuse,
- au moins une pompe adaptée pour aspirer de l'air au travers de ladite première rainure d'aspiration,

ladite bande transporteuse comportant au moins un orifice traversant positionné au moins par moment, au droit de la première rainure d'aspiration, lors du déplacement de la bande transporteuse ; au moins un opercule étant maintenu par aspiration en contact d'une face de la bande transporteuse opposée à la face principale, lorsque l'orifice est en face de la première rainure d'aspiration,
caractérisé en ce que le profilé de glissement comporte au moins une deuxième rainure d'aspiration alignée et dans le prolongement de la première rainure d'aspiration, la deuxième rainure d'aspiration débouchant au droit d'une face principale de la bande transporteuse, ladite au moins une pompe étant adaptée pour aspirer de l'air au travers de ladite la deuxième rainure d'aspiration. Avantageusement, l'aspiration peut être mise en œuvre ou arrêtée indépendamment dans la première rainure d'aspiration ou dans la deuxième rainure d'aspiration de manière à faciliter le transport d'opercule le long de la première rainure d'aspiration et la dépose d'opercules le long de la deuxième rainure d'aspiration.

Selon un mode de réalisation, la même pompe est utilisée pour aspirer de l'air au travers de la première rainure d'aspiration et au travers de la deuxième rainure d'aspiration. Selon un autre mode de réalisation, deux pompes différentes sont utilisées pour aspirer de l'air au travers de la première rainure d'aspiration et au travers de la deuxième rainure d'aspiration.

Les caractéristiques exposées dans les paragraphes suivants peuvent, optionnellement, être mises en œuvre. Elles peuvent être mises en œuvre indépendamment les unes des autres ou en combinaison les unes avec les autres :
- La au moins une bande transporteuse comporte au moins un ensemble de plusieurs orifices groupés et alignés selon la direction longitudinale, et l'entraxe entre les orifices les plus éloignés d'un ensemble d'orifices étant supérieur à l'épaisseur de la paroi entre la première rainure d'aspiration et la deuxième rainure d'aspiration.
- la au moins une bande transporteuse comporte au moins un orifice oblong s'étendant selon la direction longitudinale, l'orifice oblong ayant une première extrémité et une deuxième extrémité opposée selon la direction longitudinale ; le profilé de glissement comportant une paroi entre la première rainure d'aspiration et la deuxième rainure d'aspiration, et dans lequel l'entraxe entre la première et la deuxième extrémité de l'orifice oblong est supérieur à l'épaisseur de la paroi entre la première rainure d'aspiration et la deuxième rainure d'aspiration.

Le profilé de glissement comporte au moins une première rainure d'aspiration supplémentaire parallèle à la première rainure d'aspiration, et la bande transporteuse comporte au moins un orifice supplémentaire, l'au moins un orifice supplémentaire de la bande transporteuse étant agencée au moins par moment, au droit de ladite première rainure d'aspiration supplémentaire, lors du déplacement de la bande transporteuse.

Avantageusement, l'utilisation de deux rainures d'aspiration permet d'améliorer la fixation des opercules contre la bande transporteuse.

Le convoyeur comporte plusieurs profilés de glissement et plusieurs bandes transporteuses s'étendant selon la direction longitudinale, les profilés de glissement étant parallèles les uns aux autres et disposés les uns à côté des autres selon une direction transversale, les bandes transporteuses étant parallèles les unes aux autres et disposées les unes à côté des autres selon une direction transversale.

La bande transporteuse est réalisée en inox.

Le au moins un profilé de glissement est agencé entre une partie horizontale supérieure de la au moins une bande transporteuse et une partie horizontale inférieure de la au moins une bande transporteuse.

Le convoyeur comporte en outre un magasin de stockage d'opercules parmi un premier magasin de stockage d'opercules et un deuxième magasin de stockage d'opercules, le premier magasin de stockage d'opercules et le deuxième magasin de stockage d'opercules étant adaptés pour être montés de façon amovible au châssis, le premier magasin de stockage d'opercules comporte une plaque comprenant des ouvertures de distribution ayant un premier diamètre, le deuxième magasin de stockage d'opercules comportant une plaque comprenant des ouvertures de distribution ayant un deuxième diamètre différent du premier diamètre.

Le convoyeur comporte N profilés de glissement et N bandes transporteuses, le nombre N étant un entier naturel supérieur à 1, et la plaque du premier magasin de stockage d'opercules comporte N ouvertures de distribution, la plaque du deuxième magasin de stockage d'opercules comporte N/2 ouvertures de distribution.

Le convoyeur comporte un dispositif de dépilage des opercules apte à transférer les opercules du magasin de stockage d'opercules à la au moins une bande transporteuse, le dispositif de dépilage comportant :
- au moins deux organes de préhension montés rotatifs par rapport à un axe vertical agencé entre les deux organes de préhension,
- un dispositif d'entrainement en rotation propre à faire pivoter les organes de préhension autour de l'axe vertical ;
- les organes de préhension étant montés mobiles en translation verticalement,
- un dispositif d'entrainement en translation propre à entrainer les organes de préhension en translation entre une position haute de prise/dépose d'opercules et une position basse de transfert des opercules.

Le magasin de stockage d'opercules est fixé au châssis dans le prolongement des profilés de glissement selon la direction longitudinale, et le dispositif de dépilage est positionné en-dessous du magasin de stockage d'opercules et en-dessous d'une extrémité des profilés de glissement, l'axe vertical étant agencé entre une extrémité des profilés de glissement et le magasin de stockage d'opercules. Avantageusement, le magasin de stockage d'opercules est situé à environ 1 mètre du sol de sorte qu'il est plus facile de le charger.

Le profilé de glissement comporte au moins une rainure d'aspiration de déchargement, alignée avec la première rainure d'aspiration, la rainure d'aspiration de déchargement débouchant au droit d'une face principale de la bande transporteuse, ladite au moins une pompe étant adaptée pour aspirer de l'air au travers de ladite la rainure d'aspiration de déchargement ; le convoyeur comportant un dispositif de dépose d'au moins un opercule disposé au droit d'une rainure d'aspiration de déchargement, et une unité de distribution propre à commander la au moins une pompe pour aspirer de l'air dans la au moins une première rainure d'aspiration et dans la rainure d'aspiration de déchargement au cours d'une phase de transport des opercules et/ou de chargement des opercules, et pour aspirer de l'air uniquement dans la rainure d'aspiration de déchargement, au cours d'une phase de déchargement de au moins un opercule par le dispositif de dépose.

Avantageusement, l'existence de au moins deux rainures d'aspiration alignées l'un à l'autre, à savoir la première rainure d'aspiration et la rainure d'aspiration de déchargement permet de décharger les opercules sans les plier et de maintenir le plaquage des autres opercules qui sont transportés par le convoyeur. Ces autres opercules sont situés au droit des autres rainures d'aspiration référencées 56, 60,62, 64, 62',64', 62",64". L'arrêt de l'aspiration dans la rainure d'aspiration de déchargement est mis en œuvre au moment où, ou juste après l'activation de l'aspiration au travers des organes de préhension du dispositif de dépose.

Le au moins un profilé de glissement comporte au moins un passage traversant vertical, la bande transporteuse comprenant au moins un trou adjacent à l'orifice, et un dispositif de dépose d'au moins un opercule, ledit dispositif de dépose comportant au moins un organe de préhension et un dispositif d'entrainement en translation verticale dudit au moins un organe de préhension, ledit organe de préhension étant propre à traverser le au moins un passage du profilé de glissement et le trou de la bande transporteuse, lorsque le trou est agencé en face du au moins un passage pour détacher l'opercule de la bande transporteuse.

Le trou est positionné entre l'orifice et l'orifice supplémentaire, le au moins un passage est positionné entre la rainure d'aspiration et d'aspiration supplémentaire. Avantageusement, cet agencement facilite la séparation de l'opercule de la bande transporteuse, lors du déchargement.

L'invention concerne également une machine de transport et de décontamination qui comporte un convoyeur conformé selon les caractéristiques mentionnées ci-dessus et un dispositif de décontamination agencé en dessous d'une partie de la au moins une bande transporteuse, ledit dispositif de décontamination étant propre à décontaminer une face principale d'au moins un opercule fixé à la bande transporteuse.

### Brève description des figures

[Fig. 1] est une vue en perspective de côté d'un convoyeur selon la présente divulgation ;
[Fig. 2] est une vue en perspective partielle de côté d'une partie du convoyeur selon la présente divulgation ;
[Fig. 3] est une autre vue en perspective partielle de côté d'une partie du convoyeur selon la présente divulgation ;
[Fig. 4] est une vue en perspective d'une partie de la bande transporteuse du convoyeur illustré sur la figure 1 ;
[Fig. 5] est une vue en perspective de dessous d'un profilé de glissement du convoyeur illustré sur la figure 1 ;
[Fig. 6A] est une vue en perspective d'un premier magasin de stockage d'opercules du convoyeur illustré sur la figure 1 ;
[Fig. 6B] est une vue en perspective d'un deuxième magasin de stockage d'opercules du convoyeur illustré sur la figure 1 ;
[Fig. 7] est une vue en perspective d'un dispositif de dépilage qui transfert les opercules du magasin vers le convoyeur selon la présente divulgation ;
[Fig. 8] est une vue en perspective de côté d'un dispositif de dépose des opercules du convoyeur vers le contenant selon la présente divulgation ;
[Fig. 9] est une vue en perspective de côté d'une partie du convoyeur illustré sur la figure 1 dans une position de déplacement d'une bande transporteuse ;
[Fig. 10] est une vue en perspective de côté d'une partie du convoyeur illustré sur la figure 1 dans une position de dépose d'opercules ;
[Fig. 11] est une vue schématique d'une machine de transport et de décontamination d'opercules selon la présente divulgation ;
[Fig. 12] est une vue schématique d'un profilé de glissement selon la présente divulgation ; et
[Fig. 13] est une vue en perspective d'une partie d'une variante de la bande transporteuse du convoyeur.

### Description détaillée de l'invention

Dans la présente demande de brevet, les termes « horizontal », « vertical », « haut », « bas » sont à interpréter en liaison avec les positions représentées sur les figures.

Dans la présente demande de brevet, le terme « opercule » est utilisé pour désigner tous type de couvercle rigide ou souple.

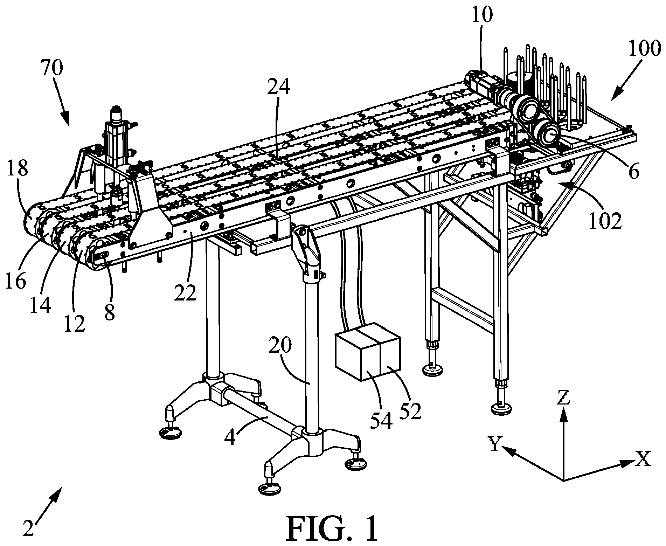

En référence à la figure 1, le convoyeur 2 selon la présente divulgation est propre à transporter des opercules. Le convoyeur 2 comporte un châssis 4, une première poulie 6 motrice et une deuxième poulie 8 libre portées par le châssis, un dispositif d'entrainement 10 en rotation de la première poulie et quatre bandes transporteuses sans fin 12, 14, 16, 18 montées autour de la première poulie et de la deuxième poulie.

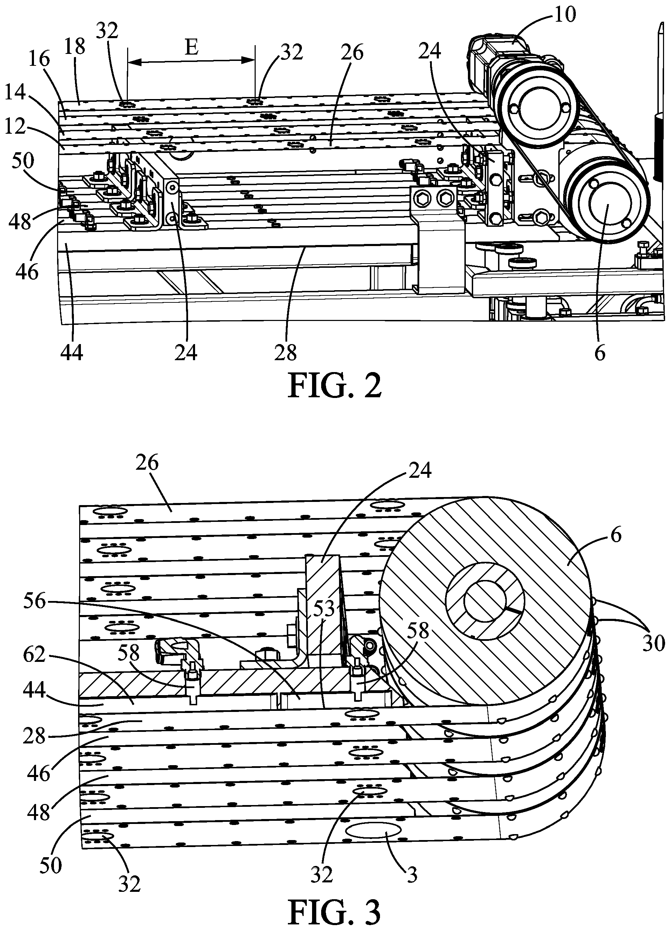

Le châssis 4 comporte des pieds 20, deux longerons 22 portés par les pieds et des traverses 24. Les longerons 22 s'étendent selon une direction longitudinale X. Les traverses 24 sont fixées à chacune de leur extrémité aux longerons. Les traverses s'étendent selon une direction transversale Y perpendiculaire à la direction longitudinale X. Les traverses sont disposées entre une partie horizontale supérieure 26 des bandes transporteuses et une partie horizontale inférieure 28 des bandes transporteuses, comme visible sur les figures 2 et 3.

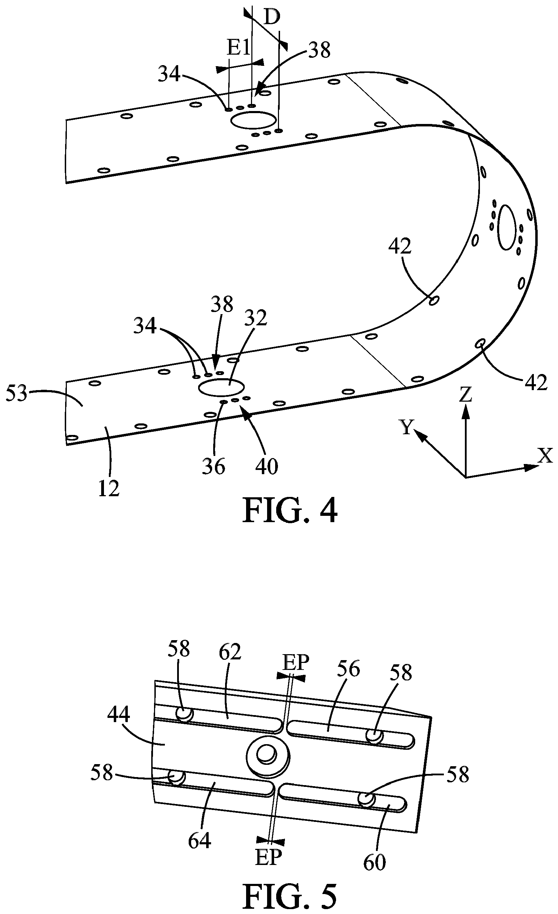

La première poulie 6 est montée en rotation autour d'un axe fixé à une extrémité des longerons 22 et perpendiculairement à ceux-ci. La première poulie 6 est située du côté du convoyeur appelé « côté chargement » dans la suite de la description. La deuxième poulie 8 est montée libre en rotation autour d'un axe fixé à l'autre extrémité des longerons 22 et perpendiculairement à ceux-ci. La deuxième poulie 8 est située du côté du convoyeur appelé « coté déchargement » dans la suite de la description. Dans le mode de réalisation représenté, la première poulie 6 et la deuxième poulie 8 comportent des picots d'entrainement 30 propres à s'insérer dans les lumières 42 de la bande visible sur la figure 4.

Le dispositif d'entrainement 10 est, par exemple, un groupe motoréducteur. Il est fixé au châssis 4.

Les quatre bandes transporteuses 12, 14, 16, 18 s'étendent selon la direction longitudinale X entre les poulies, parallèlement les unes aux autres. En référence à la figure 4, chaque bande transporteuse 12, 14, 16, 18 comporte des trous 32 traversants, des orifices 34 traversants et des orifices traversants 36 supplémentaires.

Les trous 32 sont alignés selon la direction longitudinale X et centrés par rapport aux bords longitudinaux des bandes transporteuses. Deux trous 32 adjacents sont séparés d'un entraxe E constant selon la direction longitudinale X qui correspond au pas de dépose des opercules. L'entraxe E est par exemple égal à 165 mm. La bande transporteuse a une longueur multiple de cet entraxe E.

Les orifices 34 sont alignés selon la direction longitudinale X. Ils sont adjacents aux trous. En particulier, dans le mode de réalisation représenté, les orifices 34 forment un ensemble 38 de plusieurs orifices alignés selon la direction longitudinale X et regroupés à côté d'un trou 32. Chaque ensemble d'orifices 38 est disposé à côté d'un trou. Deux ensembles d'orifices 38 adjacents sont séparés par une distance constante. L'entraxe E1 entre les orifices les plus éloignés d'un même ensemble est supérieur à l'épaisseur EP de la paroi entre une première rainure d'aspiration 56 et une deuxième rainure d'aspiration 62, comme décrit ultérieurement.

Les orifices supplémentaires 36 sont similaires aux orifices 34. Ils ne seront pas décrits en détail à nouveau. Les ensembles d'orifices supplémentaires 40 s'étendent du côté de chaque trou opposé au côté pourvu des ensembles d'orifices 38. Ainsi, chaque trou 32 est situé entre, et centré par rapport à, un ensemble d'orifices 38 et un ensemble d'orifices supplémentaires 40. La distance D selon la direction transversale Y entre un ensemble d'orifices 38 et un ensemble d'orifices supplémentaires 40 est inférieure au diamètre des opercules transportés.

En particulier, les orifices 34 et les orifices supplémentaires 36 sont inscrits à l'intérieur du diamètre du plus petit opercule à transporter dans un entraxe E1 selon la direction longitudinale X et dans une distance D selon la direction transversale Y. Préférentiellement, les bandes transporteuses sont réalisées en inox.

Dans le mode de réalisation représenté, les bords latéraux de chaque bande transporteuse 12, 13, 14, 16 comportent des lumières 42 propres à s'engager tour à tour dans les picots d'entrainement 30 des poulies lors de la rotation de celles-ci. Ces lumières 42 sont disposées à un entraxe constant multiple de l'entraxe E.

En référence aux figures 2, 3 et 5, le convoyeur 2 comporte quatre profilés de glissement 44, 46, 48, 50 portés par le châssis 4. Le convoyeur 2 comporte également une pompe 52 et un bloc distributeur 54 raccordé à la pompe 52. Le bloc distributeur 54 comporte un système de coupure de l'alimentation par électrovannes et une unité électronique de commande du système de coupure d'alimentation. Cette unité électronique de commande est par exemple constituée par un ASIC ou un processeur. Le bloc distributeur est propre à commander sélectivement l'ouverture et la fermeture de la première, la deuxième ,la troisième, la quatrième et la cinquième électrovannes afin que de l'air soit aspiré dans les rainures d'aspiration référencées 56, 60, 62, 64, 62', 64', 62",64"et dans les rainures d'aspiration de déchargement 62‴, 64‴ au cours d'une phase de chargement et /ou de transport des opercules et que de l'air soit aspiré uniquement dans les rainures d'aspiration référencées 56, 60, 62, 64, 62', 64', 62",64"au cours d'une phase de déchargement des opercules. L'air n'est pas aspiré dans les rainures d'aspiration de déchargement 62‴, 64‴, lors du déchargement des opercules, comme décrit ci-après.

La pompe 52 est une pompe à vide, par exemple de type venturi. La pompe et le bloc distributeur sont représentés schématiquement sur la figure 1.

En variante, le convoyeur comporte plusieurs pompes 52, 54.

Les profilés de glissement 44, 46, 48, 50 sont fixés en dessous des traverses 24 et sont portés par celles-ci. Elles s'étendent selon la direction longitudinale X. Une des bandes transporteuses entoure chaque profilé de glissement. Chaque profilé de glissement est agencé entre une partie horizontale supérieure 26 d'une bande transporteuse et une partie horizontale inférieure 28 d'une bande transporteuse, comme visible sur la figure 2.

Pour faciliter la compréhension, un logeron 22 n'a pas été représenté sur cette figure 2. Ainsi la face inférieure des profilés de glissement 44, 46, 48, 50 est en regard des faces principales 53 des bandes transporteuses 12,14,16,18. La face intérieure inférieure des bandes transporteuses 12, 14, 16 et 18 se retrouve au contact de la face inférieure des profilés de glissement 44, 46, 48, 50.

En référence à la figure 5, le profilé de glissement 44 comporte une première rainure d'aspiration 56 s'étendant selon la direction longitudinale X. Le profilé de glissement 44 comporte en outre une première rainure d'aspiration supplémentaire 60, au moins une deuxième rainure d'aspiration 62 et au moins une deuxième rainure d'aspiration supplémentaire 64. Ces rainures 60, 62, 64 s'étendent selon la direction longitudinale X.

La première rainure d'aspiration supplémentaire 60 est disposée parallèle à la première rainure d'aspiration 56 à une distance de celle-ci selon la direction transversale Y égale à la distance entre l'ensemble d'orifices 38 et l'ensemble d'orifices supplémentaires 40 selon la direction transversale Y.

La deuxième rainure d'aspiration 62 s'étend dans le prolongement de la première rainure d'aspiration 56. Elle est alignée à la première rainure d'aspiration 56. La deuxième rainure d'aspiration supplémentaire 64 est positionnée dans le prolongement de la première rainure d'aspiration supplémentaire 60. Elle est parallèle à la deuxième rainure d'aspiration 62.

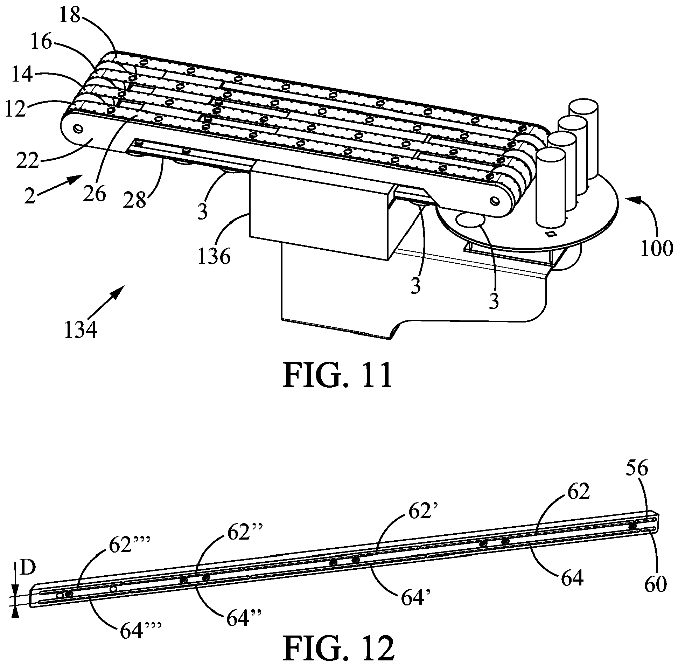

Par exemple, dans le mode de réalisation représenté sur la figure 12, le profilé de glissement 44 comporte une première rainure d'aspiration 56, une deuxième rainure d'aspiration 62, une troisième rainure d'aspiration 62', une quatrième rainure d'aspiration 62" et une cinquième rainure d'aspiration 62'" alignées les unes aux autres.

Ce profilé comporte en outre une première rainure d'aspiration supplémentaire 60, une deuxième rainure d'aspiration supplémentaire 64, une troisième rainure d'aspiration supplémentaire 64', une quatrième rainure d'extension supplémentaire 64" et une cinquième rainure d'extension supplémentaire 64‴ alignées les unes aux autres.

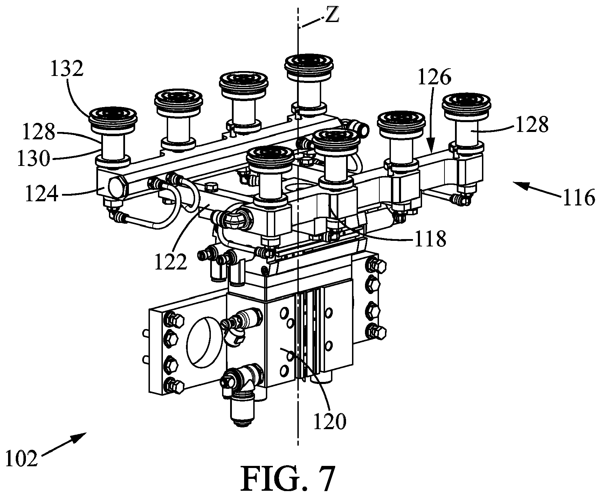

La première rainure d'aspiration 56 et la première rainure d'aspiration supplémentaire 60 forment une première zone d'aspiration. Cette première zone d'aspiration est située au-dessus du dispositif de dépilage illustré sur la figure 7.

La deuxième rainure d'aspiration 62, la deuxième rainure d'aspiration supplémentaire 64, la troisième rainure d'aspiration 62', la troisième rainure d'aspiration supplémentaire 64', la quatrième rainure d'aspiration 62"et la quatrième rainure d'aspiration supplémentaire 64" forment une deuxième zone d'aspiration. Cette deuxième zone d'aspiration est une zone de transport des opercules.

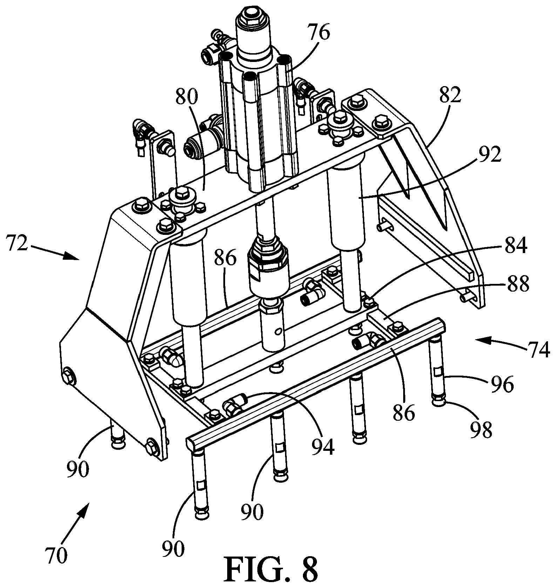

La cinquième rainure d'aspiration 62'" et la cinquième rainure d'aspiration supplémentaire 64‴ forment une troisième zone d'aspiration située au niveau du dispositif de dépose des opercules illustré sur la figure 8. Pour faciliter la compréhension de l'invention, la cinquième rainure d'aspiration 62'" et la cinquième rainure d'aspiration supplémentaire 64‴ sont aussi appelées rainures d'aspiration de déchargement.

Les rainures d'aspiration 56, 60, 62, 62', 62", 62‴, 64, 64', 64", 64‴ des profilés de glissement 44 à 50 débouchent au droit de la face principale 53 des bandes transporteuses. Les rainures d'aspiration référencées 56, 62, 62', 62", 62"'sont positionnées de telle sorte que les ensembles d'orifices 38 des bandes transporteuses soient tour à tour au droit des rainures d'aspiration référencées 56, 62, 62', 62", 62‴, lors du déplacement des bandes transporteuses. Les rainures d'aspiration référencée 60, 64, 64', 64", 64‴ sont positionnées de telle sorte que les ensembles d'orifices supplémentaires 40 des bandes transporteuses soient tour à tour au droit des rainures d'aspiration référencées 60, 64, 64, 64', 64", 64‴ lors du déplacement des bandes transporteuses. L'existence de deux ensembles de rainures d'aspiration 56, 60 parallèles permettent d'augmenter la force d'aspiration appliquée à un opercule 3 lors de son transport par une bande transporteuse. L'épaisseur EP de la paroi entre la première rainure d'aspiration 56 et la deuxième rainure d'aspiration 62 est inférieure à l'entraxe E1 entre les orifices les plus éloignés d'un ensemble d'orifices 38 de la bande transporteuse. De même, l'épaisseur EP de la paroi entre la première rainure d'aspiration supplémentaire 60 et la première rainure d'aspiration supplémentaire 64 est inférieure à l'entraxe entre les orifices les plus éloignés d'un ensemble d'orifices supplémentaires 40 de la bande transporteuse. Cette épaisseur Ep existe entre toutes les rainures d'aspiration alignées selon la direction longitudinale.

Les profilés de glissement 48 à 50 sont identiques au profilé de glissement 44. La paroi de fond de chaque rainure d'aspiration 56, 60, 62, 64 comporte une ouverture 58 connectée au bloc distributeur 54. Les ouvertures 58 des rainures d'aspiration 56 sont connectées au bloc distributeur 54 par des tubes flexibles de sorte que de l'air soit aspiré par les rainures d'aspiration. Les deuxièmes rainures d'aspiration 62 des profilés de glissement sont connectées au bloc distributeur 54. L'aspiration au travers des premières rainures d'aspiration 56 peuvent être commandée indépendamment de l'aspiration au travers des deuxièmes rainures d'aspiration 62, à l'aide des électrovannes. Ainsi, il est possible d'arrêter le pompage dans les premières rainures d'aspiration 62 tout en maintenant le pompage dans les deuxièmes rainures d'aspiration 56, par exemple lors du chargement d'opercules situés en face des premières rainures d'aspiration. Ainsi, il est possible de mettre ou d'enlever l'aspiration selon le cycle du convoyeur par exemple lors d'un cycle de dépilage des opercules, lors d'un cycle de dépose d'opercules ou lors d'un cycle de chargement des opercules.

De préférence, chaque ensemble rainure d'aspiration et rainure d'aspiration supplémentaire est alimenté par une même source d'aspiration dans le but de limiter la consommation des pompes d'aspiration lors du démarrage.

Ainsi, avec le profilé illustré sur la figure 12, les premières rainures d'aspiration 56 est 60 sont raccordées à la même électrovanne du bloc distributeur, les deuxièmes rainures d'aspiration 62 et 64 sont raccordées à une autre électrovanne du bloc distributeur. Les troisièmes rainures d'aspiration 62' et 64' sont raccordées à une troisième électrovanne. Les quatrièmes rainures d'aspiration 62" et 64" sont raccordées à une quatrième électrovanne. Enfin, les cinquièmes rainures d'aspiration 62'" et 64‴ sont raccordées à une cinquième électrovanne. Lorsque le convoyeur 2 présente une grande dimension selon la direction longitudinale X, chaque profilé de glissement peut comporter plusieurs rainures d'aspiration 62 les unes dans le prolongement des autres et éventuellement plusieurs rainures d'aspiration supplémentaire 64 également dans le prolongement les unes des autres.

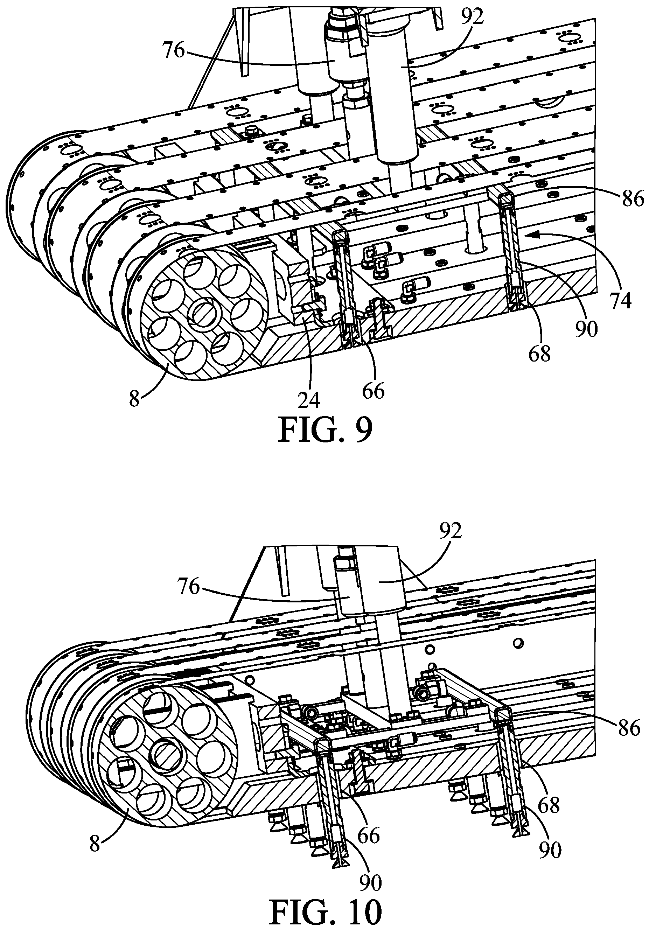

En référence aux figures 9 et 10, chaque profilé de glissement 44, 46, 48, 50 comporte en outre deux passages 66, 68 traversants verticaux situés du côté déchargement du convoyeur. Les passages 66, 68 sont positionnés entre la deuxième rainure d'aspiration 62 et la deuxième rainure d'aspiration supplémentaire 64. En particulier, les passages 66 sont centrés selon la direction transversale Y. Le diamètre des passages 66, 68 est sensiblement égal au diamètre des trous 32 des bandes transporteuses. Les passages 66, 68 sont positionnés sur les profilés de glissement de telle sorte que les trous 32 des bandes transporteuses 12, 14, 16, 18 soient tour à tour au droit des passages 66, 68, lors du déplacement des bande transporteuse. En particulier, l'entraxe entre les deux passages 66, 68 est égal à l'entraxe E entre deux trous 32 consécutifs des bandes transporteuses.

En référence aux figures 1 et 8, le convoyeur comporte en outre un dispositif 70 de dépose d'opercules propre à décharger les opercules en contact avec les bandes transporteuses et à les déposer sur des supports tels que des contenants. Le dispositif de dépose 70 est fixé au châssis 4 du côté déchargement du convoyeur. En référence à la figure 8, le dispositif de dépose 70 comporte un support 72, un ensemble de préhension 74 mobile en translation verticale par rapport au support, un dispositif d'entrainement en translation verticale 76 propre à entrainer en déplacement l'ensemble de préhension par rapport au support.

Le support 72 comprend une plaque transversale 80 et deux bras de support 82 fixés chacun à une extrémité de la plaque transversale et aux longerons 22. Les bras de support 82 sont aptes à porter la plaque transversale 80 au-dessus du convoyeur. L'ensemble de préhension 74 est porté par le support 72. Il comporte une latte centrale 84, deux conduites transversales 86 fixées à la latte centrale par des barres 88 et huit organes de préhension 90 fixés aux conduites 86. Les conduites 86 sont séparées l'une de l'autre d'une distance égale à la distance entre deux passages 66, 68 d'un même profilé de glissement. La latte centrale 84 est fixée à la plaque transversale 80 du support par deux dispositifs de guidage colonne/douille. Le dispositif d'entrainement en translation verticale 76 est fixé sur la plaque transversale 80. Il est propre à déplacer l'ensemble de préhension 74 par rapport au support 72 entre une position de repos et une position de déchargement des opercules des bandes transporteuses.

Les conduites 86 comportent des raccordements 94 connectées à soit à une nouvelle électrovanne du bloc distributeur 54 soit à une nouvelle pompe.

Chaque organe de préhension 90 comprend une portion de canal 96 ayant une extrémité en communication avec une conduite 86 et une extrémité opposée pourvue d'une ventouse 98. Les ventouses 98 ainsi que la force d'aspiration de l'air provenant du bloc distributeur 54 via une conduite permettent de maintenir les opercules 3 contre les organes de préhension 90 après leur retrait des bandes transporteuses. Les organes de préhension 90 sont séparés les uns des autres selon la direction longitudinale X d'une distance égale à l'entraxe E qui correspond à la distance entre les passages 66, 68 de deux profilés de glissement 44 à 50 adjacents. Les organes de préhension 90 sont séparés les uns des autres selon la direction transversale Y au même entraxe que l'entraxe entre les profilés 44 à 50. La dimension des organes de préhension 90 est inférieure à la dimension des passages 66, 68 des profilés de glissement.

En position de repos illustrée sur la figure 9, l'ensemble de préhension 74 est agencé entre une partie horizontale supérieure 26 des bandes transporteuses et une partie horizontale inférieure 28 des bandes transporteuses. Le bout des organes de préhension 90 est agencé dans les passages 66, 68 des profilés de glissement.

En position de déchargement, illustrée sur la figure 10, les trous 32 des bandes transporteuses 12, 14, 16, 18 sont en face des passages 66, 68 et le dispositif d'entrainement 76 déplace l'ensemble de préhension 74 vers le bas pour retirer les opercules 3 des bandes transporteuses 12, 14, 16, 18 et les déposer sur un autre support tel que des contenants.

La course de déplacement vertical des organes de préhension 90 est inférieure à la distance entre la partie horizontale supérieure de bande transporteuse et la partie horizontalement inférieure de bande transporteuse.

En référence à la figure 1, le convoyeur 2 comprend en outre au moins un magasin 100 de stockage d'opercules et un dispositif de dépilage 102 apte à transférer les opercules du magasin de stockage d'opercules au convoyeur. Le magasin 100 de stockage d'opercules est fixé au châssis 4 du côté chargement dans le prolongement des profilés de glissement 44 à 50 selon la direction longitudinale X.

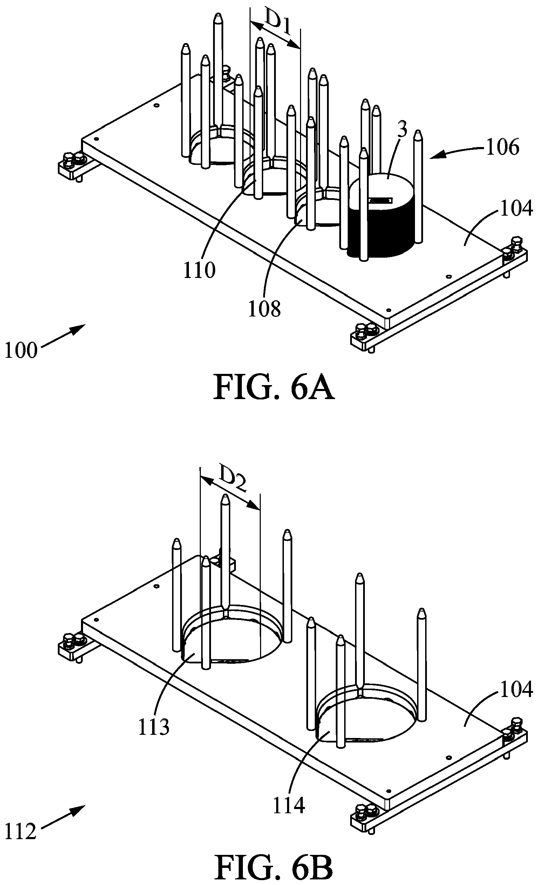

En référence aux figures 6A et 6B, le magasin 100 de stockage d'opercules comporte une plaque 104 pourvue d'une rangée de chargeurs 106. Dans le mode de réalisation représenté, la plaque 104 comporte quatre chargeurs. Chaque chargeur 106 est agencé dans l'alignement d'une bande transporteuse. Chaque chargeur 106 comporte une ouverture de distribution 108 formée dans la plaque et un guide 110 fixé autour de chaque ouverture de distribution pour maintenir ensemble l'empilement des opercules 3. Des têtes de vis non représentées sont fixées sur les bords verticaux de chaque ouverture de distribution 108 pour retenir par les bords les opercules 3 empilés sur l'ouverture. Dans le mode réalisation représenté à titre d'exemple, les guides 110 sont chacun constitués par quatre tiges verticales réparties et fixées autour des ouvertures de distribution.

Avantageusement, le convoyeur 2 comprend au moins deux magasins 100, 112 de stockage d'opercules fixés de manière amovible au châssis 4. Les magasins 100, 112 de stockage d'opercules sont chacun adaptés au stockage d'opercules de tailles différentes. Ils sont interchangeables en fonction de la taille des opercules à transporter. Ainsi, le convoyeur 2 peut comporter un premier magasin 100 de stockage d'opercules ayant des ouvertures de distribution présentant un premier diamètre D1 et un deuxième magasin 112 de stockage d'opercules ayant des ouvertures de distribution présentant un deuxième diamètre D2 différent du premier diamètre D1. Par exemple, le premier magasin est propre à stocker des opercules 3 de diamètre maximal de 95 millimètres et le deuxième magasin est propre à stocker des opercules ayant un diamètre compris entre 96 millimètres à 150 Lorsque le diamètre des opercules 3 est largement supérieur à la largeur d'une bande transporteuse, le deuxième magasin 112 de stockage d'opercules peut comporter uniquement deux chargeurs 106 et donc uniquement deux ouvertures de distribution 108.

Les ouvertures de distribution 108 sont alors disposées dans le prolongement soit de la première bande et de la troisième bande, soit de la deuxième bande et de la quatrième bande.

Dans ce cas, de façon générale, si le convoyeur possède un nombre n de bandes transporteuses, le premier magasin de stockage 100 comporte un nombre n d'ouvertures de distribution et le deuxième magasin de stockage 112 comporte un nombre n/2 d'ouvertures de distribution.

Le dispositif de dépilage 102 est positionné en-dessous du magasin de stockage et en-dessous d'une partie d'extrémité des profilés de glissement 44 à 50 du côté chargement. En référence à la figure 7, le dispositif de dépilage 102 comporte un ensemble de préhension 116, un dispositif d'entrainement en rotation 118, un dispositif d'entrainement en translation verticale 120.

L'ensemble de préhension 116 comporte un bâti 122, deux conduites transversales 124, 126 fixées au bâti parallèlement l'une à l'autre, et des organes de préhension 128 fixés sur une conduite et en communication fluidique avec celle-ci. Le nombre d'organes de préhension monté sur chaque conduite est de préférence égal au nombre de chargeurs du magasin de stockage d'opercules fixé au bâti. Dans le mode de réalisation représenté, quatre organes de préhension 128 sont fixés sur la première conduite 124 et en communication avec celle-ci. Les quatre organes de préhension sont alignés le long d'une première rangée. Quatre organes de préhension sont fixés sur la deuxième conduite 126 et en communication avec cette dernière. Les quatre organes de préhension sont alignés le long d'une deuxième rangée.

Chaque organe de préhension 128 comporte un canal creux vertical 130 ayant une extrémité supérieure ouverte et une extrémité inférieure connectée à une pompe par un tube flexible.

La première conduite 124 est connectée à la quatrième pompe et la deuxième conduite 126 est connectée à la deuxième pompe de sorte que l'aspiration peut être mise en œuvre de manière indépendante pour les organes de préhension de la première rangée et pour les organes de préhension de la deuxième rangée. Avantageusement, les organes de préhension 128 peuvent comporter des ventouses132 propres à être fixées à l'extrémité supérieure des canaux 130. Avantageusement, des ventouses de différentes tailles peuvent être montées sur l'extrémité supérieure des canaux, la taille des ventouses étant choisie en fonction de la taille des opercules.

L'ensemble de préhension 116 est monté rotatif par rapport à un axe vertical Z-Z centré par rapport à la première et la deuxième conduites. L'axe vertical Z-Z est situé entre et à égale distance de la première et de la deuxième conduites. L'axe vertical Z-Z est également agencé entre l'extrémité des profilés de glissement et le magasin de stockage d'opercules.

Le dispositif d'entrainement en rotation118 est propre à faire pivoter l'ensemble de préhension autour de l'axe vertical Z-Z d'un demi-tour pour que les organes de préhension de la première rangée et de la deuxième rangée soient tour à tour d'une part, en dessous des ouvertures du magasin de stockage d'opercules et d'autre part, en dessous des ensembles d'orifices 38, 40 des bandes transporteuses. L'ensemble de préhension est également monté mobile en translation verticalement. Le dispositif d'entrainement en translation 120 est apte à déplacer verticalement l'ensemble de préhension 74 entre une position haute de prise/dépose d'opercules et une position basse de rotation des opercules. Les dispositifs d'entrainement en rotation et en translation sont par exemple des actionneurs électriques, des vérins pneumatiques ou hydrauliques.

En variante, le convoyeur comporte un nombre n de bande(s) transporteuse(s) et un nombre n de profilé(s) de glissement avec un nombre n différent de quatre. Par exemple, le nombre n peut être égal à un, deux ou trois ou être supérieur à quatre. Le nombre de pompes ou de zone d'aspiration peut être différent. Par exemple, deux pompes différentes peuvent être connectées l'une à la rainure d'aspiration, et l'autre à la rainure d'aspiration supplémentaire.

En variante, le convoyeur comporte également un dispositif d'entrainement en rotation de la deuxième poulie.

En variante, la première et la deuxième poulies peuvent être remplacées par un assemblage de plusieurs poulies qui peuvent être entrainées en rotation indépendamment les unes des autres pour pouvoir réduire le nombre d'opercules à transporter si besoin.

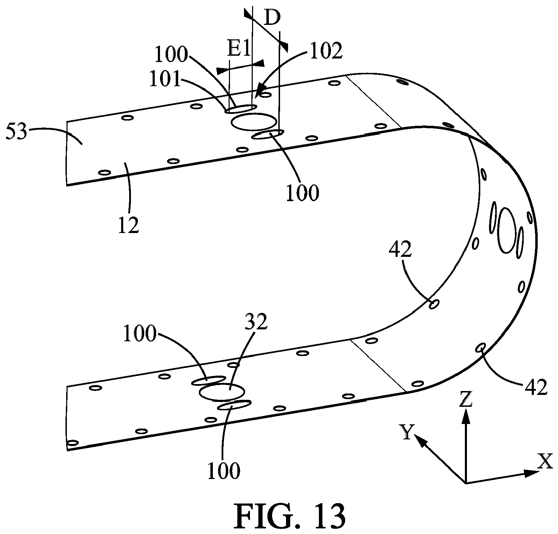

Selon une variante illustrée sur la figure 13, chaque ensemble d'orifices et chaque ensemble d'orifices supplémentaires est remplacé par un orifice de forme oblongue 100 s'étendant selon la direction longitudinale X. Les orifices de forme oblongue 100 sont disposés de part et d'autre de chaque trou 32. Ils sont alignés selon la direction longitudinale. L'orifice oblong ayant une première extrémité 101 et une deuxième extrémité opposée 102 selon la direction longitudinale X. L'entraxe E1 entre la première extrémité 101 et la deuxième extrémité 102 est supérieur à l'épaisseur EP de la paroi entre la première rainure d'aspiration 56 et la deuxième rainure d'aspiration 62.

En variante, les profilés de glissement 44 à 50 comportent un unique passage 66 et le dispositif de dépose 70 ne comporte qu'une conduite 86.

Selon une variante moins avantageuse, les rainures des profilés de glissement sont remplacées par des conduits s'étendant longitudinalement, les conduit comportant plusieurs ouvertures alignées débouchant sur une face extérieure des profilés de glissement.

En référence à la figure 11, l'invention concerne également une machine134 de transport et de décontamination qui comporte un convoyeur 2 comme décrit ci-dessus, et un dispositif de décontamination 136 agencé en dessous ou autour de la partie horizontale inférieure 28 des bandes transporteuses. Le dispositif de décontamination est propre à décontaminer une face principale des opercules, cette face principale étant opposée à la face principale 53 qui est au droit d'un profilé de glissement. Le dispositif de décontamination est par exemple un dispositif de décontamination à ultra-violet, un dispositif de décontamination à lumière pulsée ou un dispositif de décontamination au peroxyde.

En fonctionnement, les chargeurs 106 du magasin de stockage d'opercules comportent des opercules 3 empilés les uns sur les autres. Les organes de préhension 128 du dispositif de dépilage 102 sont déplacés en translation vers une position haute de prise/dépose d'opercules. Les organes de préhension 128 de la première rangée sont disposés sous les ouvertures de distribution 108. La pompe raccordée au dispositif de dépilage aspire les opercules stockés dans les chargeurs. Les organes de préhension 128 de la première et de la deuxième rangées sont déplacés verticalement vers une position basse. Ils pivotent d'un demi-tour, puis ils sont déplacés verticalement vers une position haute. Les organes de préhension 128 de la première rangée sont alors plaqués en face des bandes transporteuses 12,14,16,18. La pompe connectée aux organes de préhension de la première rangée est stoppée. L'aspiration est générée au travers de la première rainure d'aspiration 56 et de la première rainure d'aspiration supplémentaire 60. Les opercules 3 sont transférés par aspiration du dispositif de dépilage 102 aux bandes transporteuses 12, 14, 16, 18 grâce à l'aspiration provenant des rainures d'aspiration et passant par les ensembles d'orifices 38, 40. Lorsque les opercules 3 sont fixés par aspiration aux bandes transporteuses, les bandes transporteuses et les opercules 3 se déplacent selon la direction longitudinale X vers le côté déchargement. Les parties de bandes transporteuses pourvues d'opercules se retrouvent en regard des deuxièmes rainures d'aspiration 62 et des deuxièmes rainures d'aspiration supplémentaires 64. Elles sont ensuite transportées d'une rainure d'aspiration à l'autre jusqu' à la zone de dépose correspondant aux cinquièmes rainures d'aspiration 62‴ et 64‴. Lorsque les opercules arrivent au droit du passage 66 ou 68, l'aspiration au travers des cinquièmes rainures d'aspiration 62'" et aux cinquièmes rainures d'aspiration supplémentaires'" 64 est stoppée. L'aspiration au travers des organes de préhension 90 du dispositif de dépose 70 est mise en fonctionnement. Les ventouses 98 chargent les opercules. L'aspiration est maintenue au travers des rainures d'aspiration référencées 62, 64, 62', 64', 62", 64", 62‴, 64"" qui correspondent à une zone de transfert et de décontamination des opercules. Les organes de préhension 90 sont déplacés verticalement vers le bas, comme illustré sur la figure 10 . Les organes de préhension 90 déposent les opercules sur un support tel que par exemple sur des contenants.

Selon une variante non représentée, le profilé de glissement comportant uniquement une première rainure d'aspiration s'étendant selon la direction longitudinale et une deuxième rainure d'aspiration alignée et dans le prolongement de la première rainure d'aspiration. Le dispositif de dépose est disposé au droit de la deuxième rainure d'aspiration, et le bloc de distribution est propre à commander la pompe pour aspirer de l'air dans la première rainure d'aspiration et dans la deuxième rainure d'aspiration au cours de la phase de chargement de la bande transporteuse et/ou de la phase de transport des opercules. Et le bloc de distribution est propre à commander la pompe pour aspirer de l'air uniquement dans la première rainure d'aspiration au cours de la phase de déchargement des opercules par le dispositif de dépose. Aucun air n'est aspiré au travers de la deuxième rainure d'aspiration au cours de la phase de déchargement des opercules. La deuxième rainure d'aspiration constitue une rainure d'aspiration de déchargement.

## Revendications

1. Convoyeur (2) de transport d'opercules (3) comprenant :
- un châssis (4),
- au moins une première poulie (6) et une deuxième poulie (8) portées par le châssis,
- un dispositif (10) d'entrainement en rotation propre à entrainer en rotation au moins la première poulie,
- au moins une bande transporteuse (12,14,16,18) sans fin montées entre la première poulie (6) et la deuxième poulie (8), la bande transporteuse s'étendant selon une direction longitudinale (X), la bande transporteuse (12,14,16,18) étant entrainée en déplacement par la première poulie,
- au moins un profilé de glissement (44, 46, 48, 50) porté par le châssis, le profilé de glissement comportant une première rainure d'aspiration (56) s'étendant selon la direction longitudinale la première rainure d'aspiration débouchant au droit d'une face principale (53) de la bande transporteuse,
- au moins une pompe (52) adaptée pour aspirer de l'air au travers de ladite première rainure d'aspiration,
ladite bande transporteuse (12,14,16,18) comportant au moins un orifice (34) traversant positionné au moins par moment, au droit de la première rainure d'aspiration (56), lors du déplacement de la bande transporteuse ; au moins un opercule (3) étant maintenu par aspiration en contact d'une face de la bande transporteuse opposée à la face principale, lorsque l'orifice (34) est en face de la première rainure d'aspiration (56),
**caractérisé en ce que** le profilé de glissement comporte au moins une deuxième rainure d'aspiration (62) alignée et dans le prolongement de la première rainure d'aspiration, la deuxième rainure d'aspiration débouchant au droit d'une face principale (53) de la bande transporteuse, ladite au moins une pompe (52) étant adaptée pour aspirer de l'air au travers de ladite la deuxième rainure d'aspiration.

2. Convoyeur (2) selon la revendication 1, dans lequel la au moins une bande transporteuse (12,14,16,18) comporte au moins un ensemble (38) de plusieurs orifices groupés et alignés selon la direction longitudinale (X), le profilé de glissement comportant une paroi entre la première rainure d'aspiration (56) et la deuxième rainure d'aspiration (62), et dans lequel l'entraxe (E1) entre les orifices les plus éloignés d'un ensemble d'orifices est supérieur à l'épaisseur de la paroi entre la première rainure d'aspiration (56) et la deuxième rainure d'aspiration (62).

3. Convoyeur (2) selon la revendication 1, dans lequel la au moins une bande transporteuse (12,14,16,18) comporte au moins un orifice oblong (100) s'étendant selon la direction longitudinale (X), l'orifice oblong (100) ayant une première extrémité (101) et une deuxième extrémité opposée selon la direction longitudinale ; le profilé de glissement comportant une paroi entre la première rainure d'aspiration (56) et la deuxième rainure d'aspiration (62), et dans lequel l'entraxe (E1) entre la première (101) et la deuxième (102) extrémité de l'orifice oblong (100) est supérieur à l'épaisseur (EP) de la paroi entre la première rainure d'aspiration (56) et la deuxième rainure d'aspiration (62).

4. Convoyeur (2) selon l'une quelconque des revendications 1 à 3, dans lequel le profilé de glissement (44, 46, 48, 50) comporte au moins une première rainure d'aspiration supplémentaire (60) parallèle à la première rainure d'aspiration (56), et dans lequel la bande transporteuse (12,14,16,18) comporte au moins un orifice supplémentaire (36), l'au moins un orifice supplémentaire de la bande transporteuse étant agencée au moins par moment, au droit de ladite première rainure d'aspiration supplémentaire, lors du déplacement de la bande transporteuse.

5. Convoyeur (2) selon l'une quelconque des revendications 1 à 4, qui comporte plusieurs profilés de glissement (44, 46, 48, 50) et plusieurs bandes transporteuses (12,14,16,18) s'étendant selon la direction longitudinale (X), les profilés de glissement (44, 46, 48, 50) étant parallèles les uns aux autres et disposés les uns à côté des autres selon une direction transversale (Y), les bandes transporteuses étant parallèles les unes aux autres et disposées les unes à côté des autres selon une direction transversale (Y).

6. Convoyeur (2) selon l'une quelconque des revendications 1 à 5, dans lequel la bande transporteuse (12,14,16,18) est réalisée en inox.

7. Convoyeur (2) selon l'une quelconque des revendications 1 à 6, dans lequel le au moins un profilé de glissement (44, 46, 48, 50) est agencé entre une partie horizontale supérieure (26) de la au moins une bande transporteuse et une partie horizontale inférieure (28) de la au moins une bande transporteuse.

8. Convoyeur (2) selon l'une quelconque des revendications 1 à 7, qui comporte en outre un magasin (100, 112) de stockage d'opercules parmi un premier magasin (100) de stockage d'opercules et un deuxième magasin (112) de stockage d'opercules, le premier magasin de stockage d'opercules et le deuxième magasin de stockage d'opercules étant adaptés pour être montés de façon amovible au châssis (4), le premier magasin(100) de stockage d'opercules comporte une plaque (104) comprenant des ouvertures de distribution (108) ayant un premier diamètre (D1), le deuxième magasin (112) de stockage d'opercules comportant une plaque (104) comprenant des ouvertures de distribution (108) ayant un deuxième diamètre (D2) différent du premier diamètre.

9. Convoyeur (2) selon la revendication 8, qui comporte N profilés de glissement (44, 46, 48, 50) et N bandes transporteuses (12,14,16,18), le nombre N étant un entier naturel supérieur à 1, et dans lequel la plaque (104) du premier magasin (100) de stockage d'opercules comporte N ouvertures de distribution (108), la plaque (104) du deuxième magasin de stockage (112) d'opercules comporte N/2 ouvertures de distribution (113,114).

10. Convoyeur (2) selon l'une quelconque des revendications 1 à 9, qui comporte un dispositif de dépilage (102) des opercules apte à transférer les opercules (3) du magasin de stockage d'opercules (100, 112) à la au moins une bande transporteuse (12, 14, 16, 18), le dispositif de dépilage (102) comportant :
- au moins deux organes de préhension (128) montés rotatifs par rapport à un axe vertical (Z°) agencé entre les deux organes de préhension (128), ,
- un dispositif d'entrainement en rotation (118) propre à faire pivoter les organes de préhension autour de l'axe vertical (Z) ;
- les organes de préhension (128) étant montés mobiles en translation verticalement,
- un dispositif d'entrainement en translation (120) propre à entrainer les organes de préhension (128) en translation entre une position haute de prise/dépose d'opercules et une position basse de transfert des opercules.

11. Convoyeur (2) selon la revendication 10, dans lequel le magasin (100, 112) de stockage d'opercules est fixé au châssis (4) dans le prolongement des profilés de glissement (44,46,48,50) selon la direction longitudinale (X), et dans lequel le dispositif de dépilage (102) est positionné en-dessous du magasin (100, 112) de stockage d'opercules et en-dessous d'une extrémité des profilés de glissement (44,46,48,50), l'axe vertical (Z) étant agencé entre une extrémité des profilés de glissement (44,46,48,50) et le magasin (100, 112) de stockage d'opercules.

12. Convoyeur (2) selon l'une quelconque des revendications 1 à 11, dans lequel le profilé de glissement comporte au moins une rainure d'aspiration de déchargement (62,62‴), alignée avec la première rainure d'aspiration, la rainure d'aspiration de déchargement débouchant au droit d'une face principale (53) de la bande transporteuse, ladite au moins une pompe (52) étant adaptée pour aspirer de l'air au travers de ladite la rainure d'aspiration de déchargement ; le convoyeur comportant un dispositif de dépose (70) d'au moins un opercule disposé au droit d'une rainure d'aspiration de déchargement (62,62‴), et une unité de distribution (54) propre à commander la au moins une pompe (52) pour aspirer de l'air dans la au moins une première rainure d'aspiration (56) et dans la rainure d'aspiration de déchargement (62,62‴) au cours d'une phase de transport des opercules et/ou de chargement des opercules, et pour aspirer de l'air uniquement dans la rainure d'aspiration de déchargement (62,62‴), au cours d'une phase de déchargement de au moins un opercule par le dispositif de dépose.

13. Convoyeur (2) selon la revendications 12, dans lequel le au moins un profilé de glissement (44, 46, 48, 50) comporte au moins un passage (66, 68) traversant vertical, la bande transporteuse (12,14,16,18) comprenant au moins un trou (32) adjacent à l'orifice (34), et dans lequel ledit dispositif de dépose comporte au moins un organe de préhension (90) et un dispositif (76) d'entrainement en translation verticale dudit au moins un organe de préhension, ledit organe de préhension (90) étant propre à traverser le au moins un passage (66, 68) du profilé de glissement et le trou (32) de la bande transporteuse, lorsque le trou (32) est agencé en face du au moins un passage pour détacher l'opercule (3) de la bande transporteuse (12,14,16,18).

14. Convoyeur (2) selon la combinaison des revendications 4 et 13, dans lequel le trou (32) est positionné entre l'orifice (34) et l'orifice supplémentaire (36), le au moins un passage (66) est positionné entre la première rainure d'aspiration (56) et la première rainure d'aspiration supplémentaire (60).

15. Machine (134) de transport et de décontamination qui comporte un convoyeur (2) selon l'une quelconque des revendications 1 à 14, et un dispositif de décontamination (136) agencé en dessous d'une partie de la au moins une bande transporteuse (12,14,16,18), ledit dispositif de décontamination (136) étant propre à décontaminer une face principale d'au moins un opercule fixé à la bande transporteuse.
